(19) Europäisches Patentamt
European Patent Office
Office européen des brevets

(11) Numéro de publication: 0 341 113
A1

(12) DEMANDE DE BREVET EUROPEEN

(21) Numéro de dépôt: 89401086.7

(22) Date de dépôt: 19.04.89

(51) Int. Cl.[4]: C 07 C 39/27
C 07 C 37/60, C 01 B 21/22

(30) Priorité: 02.05.88 FR 8805848

(43) Date de publication de la demande:
08.11.89 Bulletin 89/45

(84) Etats contractants désignés:
AT BE CH DE ES FR GB GR IT LI LU NL SE

(71) Demandeur: RHONE-POULENC CHIMIE
25, quai Paul Doumer
F-92408 Courbevoie Cédex (FR)

(72) Inventeur: Gubelmann, Michel
39, boulevard des Belges
F-69006 Lyon (FR)

Tirel, Philippe-Jean
40, boulevard Kennedy
F-69600 Oullins (FR)

(74) Mandataire: Le Pennec, Magali et al
Rhone-Poulenc Chimie Service Brevets Chimie 25 Quai Paul Doumer
F-92408 Courbevoie (FR)

(54) **Procédé de préparation d'halogénophénols.**

(57) La présente invention concerne un procédé de préparation d'halogénophénols par mise en contact d'un halogénobenzène avec du protoxyde d'azote sur une zéolithe acidifiée.

EP 0 341 113 A1

**Description**

## PROCEDE DE PREPARATIONS D'HALOGENOPHENOLS

La présente invention concerne un procédé de préparation d'halogénophénols ; elle concerne plus particulièrement un procédé d'hydroxylation directe d'halogénobenzènes.

Il est connu depuis longtemps divers procédés de synthèses d'halogénophenols et tout particulièrement de synthèse de fluoro phénols.

Il a par exemple été décrit dans le brevet DE 3430554 un procédé qui consiste à partir de bromofluorobenzène à effectuer une hydrolyse par l'hydroxyde de baryum sur un catalyseur à base de cuivre. Les rendements de ce type de raction sont excellents, mais le bromofluorobenzène est une matière première onéreuse dont la synthèse est difficile, car sa préparation exige deux étapes de synthèse et provoque la corrosion des réacteurs. L'industrie cherche donc depuis longtemps à s'affranchir d'une telle matière première.

Il est aussi connu d'après un article de BERGMANN, paru dans le Journal of the American Chemical Society 78, 6037 en 1956, un procédé de fluoration d'hydroxyanilines par diazotation en présence d'acide tétrafluoroborique, de catalyseur au cuivre dans l'acétone. Cette technique, même si elle part de matière première peu onéreuse, est difficile à mettre en oeuvre.

En effet, la manipulation et le coût de l'acide tétrafluoroborique rendent ce procédé difficile à exploiter dans toute industrie chimique non avertie quant aux problèmes de sécurité liés à la manipulation des mélanges HF - $BF_3$.

Il est encore connu d'après un article de FINGER G.C. et Coll paru dans le Journal of the American Chemical Society, 81, 94-7 en 1959 de réaliser une réaction de diazotation sur la parafluoroaniline dans l'acide sulfurique.

La parafluoroaniline, comme les bromofluorobenzènes, n'est pas une matière première économique que l'industrie aime utiliser pour les synthèses de gros volumes.

Le coût des matières premières et la difficulté de mener à bien des réactions de diazotation dans des conditions de sécurité strictes fait que l'industrie chimique est toujours à la recherche de procédés de synthèses simples et économiques pour préparer les halogénophénols.

A partir d'halogénobenzènes, les chimistes ont cherché depuis longtemps à introduire directement sur le noyau benzénique le groupement hydroxyle désiré. Cette chimie a longtemps échoué. La seule publication décrivant l'introduction directe d'un groupe hydroxyle sur un noyau benzénique consiste en un article de IWAMOTO paru dans le Journal of Physical Chemistry, 87, 6, 1983.

Cette réaction d'hydroxylation du benzène est réalisée par le protoxyde d'azote ($N_2O$) en présence d'un catalyseur à base d'un oxyde d'un métal des groupes V ou VI de la classification périodique.

L'oxyde de vanadium est l'oxyde préféré parmi les oxydes des métaux des groupes V et VI de la classification. Il est préférable d'utiliser cet oxyde disposé sur un support à base de silice en quantité pondérale comprise entre 1 et 10 % par rapport au support. Le support est constitué de préférence de silice, l'alumine provoquant majoritairement la formation d'un mélange d'oxydes de carbone.

Le dit procédé était intéressant mais l'utilisation de ces catalyseurs contenant les oxydes de vanadium le rendait peu attrayant pour l'industrie.

L'industrie chimique est donc toujours à la recherche d'un procédé d'hydroxylation directe du noyau benzénique sur un substrat simple et facilement disponible.

La présente invention a permis d'atteindre cet objectif.

Elle a pour objet un procédé de préparation d'halogénophénols caractérisé en ce que l'on met en présence un halogénobenzène et du protoxyde d'azote sur une zéolithe acidifiée.

La zéolithe est choisie notamment parmi les formes commerciales telles que :

. la zéolithe ZSM-5 de MOBIL-OIL dont la préparation est décrite dans le brevet US 3702886
. la zéolithe US-Y vendue par TOYO-SODA
. la zéolithe HY vendue par UNION CARBIDE CHEMICALS sous la référence LZY 82.
. la zéolithe H-Mordénite vendue par LA GRANDE PAROISSE.

On préfére utiliser la zéolithe commerciale ZSM-5.

La zéolithe a de préférence un rapport $SiO_2/Al_2O_3$ supérieur à 90, encore plus préférentiellement compris entre 90 et 500.

La zéolithe commerciale est, de préférence dans le procédé de l'invention, acidifiée par addition d'un acide inorganique choisi parmi l'acide chlorhydrique, l'acide sulfurique, l'acide nitrique, l'acide perchlorique et l'acide phosphorique ou d'un acide organique choisi parmi les acides halosulfoniques, halométhane sulfoniques, halocarboxyliques et de préférence l'acide trifluorométhanesulfonique.

Les acides organiques ne présentent aucun avantage par rapport aux acides minéraux et présentent surtout l'inconvénient d'être plus onéreux.

Selon un mode de mise en oeuvre préférée, la zéolithe est acidifiée par passage d'un volume d'acide présentant une normalité comprise entre 0,1 N et 2 N par gramme de zéolithe compris entre 10 ml/g et 100 ml/g. Ce passage peut être réalisé en une seule étape ou de préférence en plusieurs étapes successives.

Les halogénobenzènes sont choisis de préférence parmi les dérivés benzéniques halogènés, éventuellement substitués par un groupe alkyl ou alkoxy contenant un à deux atomes de carbone. Ils répondent à la formule (I).

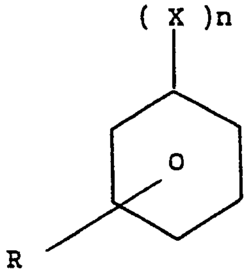

( I )

dans laquelle

. R représente l'hydrogène, un groupe alkyle ou alkoxy contenant 1 à 2 atomes de carbones.

. X représente un halogène et de préférence le chlore ou le fluor

. n est un nombre entier supérieur ou égal à 1 et inférieur ou égal à 3.

On préfère tout particulièrement utiliser le fluorobenzène

Le protoxyde d'azote est utilisé pur ou en mélange avec un gaz inerte ne contenant pas d'oxygène tel que l'azote.

L'halogéno benzène est de préférence introduit en mélange avec le protoxyde d'azote selon un rapport molaire du protoxyde d'azote par rapport à l'halogénobenzène compris entre 1 et 10.

Selon un procédé de mise en oeuvre préférentiel, on vaporise l'halogénobenzène, on le mélange avec le protoxyde d'azote selon les proportions préalablement définies et on le fait circuler sur la zéolithe. La réaction se déroule de préférence à une température comprise entre 300 et 500°C.

Les gaz de réaction contenant un mélange d' isomères des halogéno phénols sont condensés et séparés pour toute technique connue de l'homme de l'art.

Les exemples suivants donnés uniquement à titre indicatif et nullement limitatif permettent d'illustrer l'invention.

Dans les exemples, les abréviations suivantes signifient :

$$TT = \text{taux de transformation} = \frac{\text{produit transformé}}{\text{produit introduit}} \text{ en moles}$$

$$RT = \text{rendement sur produit transformé} = \frac{\text{produit désiré}}{\text{produit transformé}} \text{ en moles}$$

EXEMPLE n° 1 à 3

Préparation du catalyseur

On met en contact 10 g de zéolithe commerciale NaZSM5 avec 100 ml d'une solution d'HCl 1N à 60°C pendant 4 heures sous agitation. On laisse refroidir et on lave à l'eau permutée. On filtre le solide et on le sèche à l'étuve à 100°C.

Le lavage ci-dessus décrit est répété 4 fois. Le produit séché obtenu après le 4ème lavage est broyé.

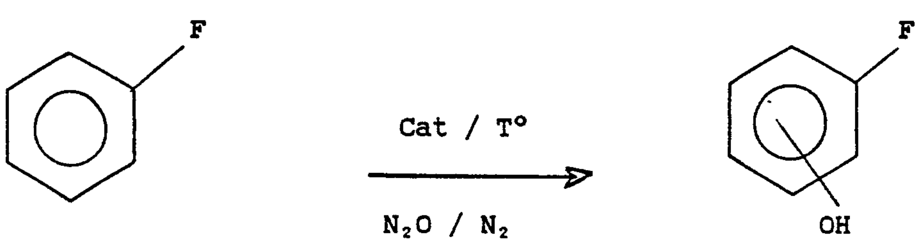

CONDITIONS EXPERIMENTALES :

| | | |
|---|---|---|
| Phase vapeur | : | continue |
| Catalyseur | : | . H Z S M 5 |
| | | . diam pores : 550 pm |
| | | . Rapport $\frac{SiO_2}{Al_2O_3}$ = 120 |
| Température | : | Essais à 350°C ; 400°C ; 450°C |
| Temps de contact | : | 1 seconde |
| Rapports molaires | : | Fluorobenzène / $N_2$ / $N_2O$ |
| | | 2 5 8 |

1,05 g de catalyseur HZSM5 ($SiO_2/Al_2O_3$ = 120) en poudre, dispersé dans 4 g de quartz en grains (< 0,8 mm) sont introduits dans un réacteur tubulaire en quartz (longueur = 16 cm, diamètre intérieur = 1,8 cm).

On ajoute ensuite un lit de 10 cm de billes de verre permettant d'homogénéiser le mélange gazeux. Le réacteur ainsi chargé est conditionné 15 heures à 350°C sous azote dans un four tubulaire.

Le catalyseur est ensuite traité pendant 30 mn à la température de réaction par 4 cm³ de fluorobenzène et 2,4 l/h d'azote.

La réaction est effectuée en continu en introduisant 1,5 cm³/h de fluorobenzène, 1,44 l/h de protoxyde d'azote et 0,9 l/h d'azote. Soit en rapport molaire :

| Fluorobenzène / | $N_2$ / | $N_2O$ |
|---|---|---|
| 2 | 5 | 8 |

Les résultats des exemples 1 à 3 sont rassemblés dans le tableau I.

EXEMPLE 4 à 6

Dans les mêmes conditions qu'à l'exemple 1, on fait varier la nature de la zéolithe et le nombre d'acidification.

| | |
|---|---|
| Température | : 400°C |
| rapport volumique | : fluorobenzène/azote/ protoxyde d'azote (2 / 5 / 8) |
| temps de contact | : 1 seconde |

Les résultats des exemples 4 à 6 sont rassemblés dans le tableau II.

TABLEAU I.

| EX | CATALYSEUR | tr (h) | T (°C) | TT(ØF) % | RT TOTAL (FØOH)% | REPARTITION ISOMERIQUE | | | RT (ØOH) % | RT % TOTAL |
|---|---|---|---|---|---|---|---|---|---|---|
| | | | | | | ORTHO | META | PARA | | |
| 1 | ZEOLITHE | 1 | 350 | 9,2 | 90,4 | 26,7 | 20,0 | 53,3 | 7,2 | 97,6 |
| 2 | $\frac{SiO_2}{Al_2O_3} = 120$ | 1 | 400 | 11,0 | 90,4 | 31,1 | 22,7 | 46,2 | 4,1 | 94,5 |
| 3 | | 1 | 450 | 15,2 | 85,3 | 24,4 | 23,7 | 51,9 | 2,7 | 88,0 |
| COMP1 | SILICALITE | 1 | 400 | 0 | 0 | - | - | - | - | - |
| COMP2 | NEANT | 1 | 400 | 0 | 0 | - | - | - | - | - |

TABLEAU II.

| EX | CATALYSEUR | tr (HR) | T (°C) | TT(ØF) % | RT TOTAL (FØOH)% | REPARTITION ISOMERIQUE % | | | RT (ØOH) % |
|---|---|---|---|---|---|---|---|---|---|
| | | | | | | ORTHO | META | PARA | |
| 4 | $SiO_2$ / $Al_2O_3$ = 120<br>4 Ech. H⁺ | 1 | 400 | 11 | 90 | 31 | 23 | 46 | 4 |
| 5 | $SiO_2$ / $Al_2O_3$ = 120<br>8 Ech. H⁺ | 1 | 400 | 14 | 91 | 28 | 23 | 49 | 1 |
| COMP3 | $SiO_2$ / $Al_2O_3$ = 40 | 1 | 400 | 4 | 31 | 36 | 28 | 36 | 68 |
| 6 | HY | 1 | 400 | 1 | 50 | NON DETERMINE | | | |

**Revendications**

1. Procédé de préparation d'halogénophénols caractérisé en ce que l'on met en présence un halogénobenzène et du protoxyde d'azote sur une zéolithe acidifiée.

2. Procédé selon revendication 1 caractérisé en ce que l'halogénobenzène répond à la formule (I)

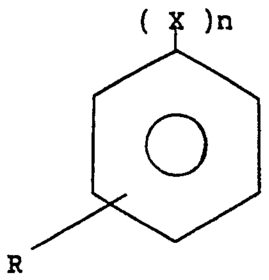

( I )

dans laquelle
. X représente un halogène
. R représente l'hydrogène, un radical alkyle ou alkoxy contenant 1 à 2 atomes de carbones.
. n est un nombre entier supérieur ou égal à 1 et inférieur ou égal à 3.

3. Procédé selon la revendication 2 caractérisé en ce que X représente le chlore ou le fluor

4. Procédé selon les revendications 2 ou 3 caractérisé en ce que le composé de formule (I) est le fluorobenzène.

5. Procédé selon la revendication 1 caractérisé en ce que la zéolithe acidifiée est choisie parmi les zéolithes HZSM-5, HY et H-Mordénite.

6. Procédé selon la revendication 1 caractérisé en ce que la zéolithe est acidifiée par un acide inorganique ou un acide organique.

7. Procédé selon la revendication 1 caractérisé en ce que le protoxyde d'azote est utilisé selon un rapport molaire calculé par rapport à l'halogénobenzène compris entre 1 et 10.

8. Procédé selon la revendication 1 caractérisé en ce que la réaction est mise en oeuvre à une température comprise entre 300 et 500° C.

Office européen des brevets

# RAPPORT DE RECHERCHE EUROPEENNE

Numero de la demande

EP 89 40 1086

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-1 547 725 (BIBB)<br>* page 1, lignes 83,84;89-93, revendications * | 1 | C 07 C 39/27<br>C 07 C 37/60<br>C 01 B 21/22 |
| A | PATENT ABSTRACTS OF JAPAN tome 10, no. 31 (C-327)(2088), 6 février 1986; & JP - A - 60 184 036 (IWAMOTO) 19-09-1985 | 1 | |
| D,A | THE JOURNAL OF PHYSICAL CHEMISTRY tome 87, no. 6, 17 mars 1983, pages 903-905; M. IWAMOTO et al.:"Catalytic Oxidation by Oxide Radical Ions. 1. One-Step Hydroxylation of Benzene to Phenol over Group 5 and 6 Oxides Supported on Silica Gel" * le document en entier * | 1 | |
| A | US-A-4 578 521 (CHANG)<br>* colonne 6, exemple 2 * | 1 | |

| DOMAINES TECHNIQUES RECHERCHES (Int. Cl.4) |
|---|
| C 07 C 39/00<br>C 07 C 37/00<br>C 01 B 21/00 |

Le présent rapport a été établi pour toutes les revendications

| Lieu de la recherche | Date d'achèvement de la recherche | Examinateur |
|---|---|---|
| BERLIN | 19-07-1989 | PROBERT C.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)